(19) **Europäisches Patentamt / European Patent Office / Office européen des brevets**

(11) **EP 4 359 379 B1**

(12) **FASCICULE DE BREVET EUROPEEN**

(45) Date de publication et mention de la délivrance du brevet:
**08.04.2026 Bulletin 2026/15**

(21) Numéro de dépôt: **22741353.1**

(22) Date de dépôt: **20.06.2022**

(51) Classification Internationale des Brevets (IPC):
**C07C 321/04** *(2006.01)*    **C07C 319/06** *(2006.01)*
**C07C 319/28** *(2006.01)*

(52) Classification Coopérative des Brevets (CPC):
(C-Sets disponibles)
**C07C 321/04; C07C 319/06; C07C 319/28**   (Cont.)

(86) Numéro de dépôt international:
**PCT/FR2022/051193**

(87) Numéro de publication internationale:
**WO 2022/269183 (29.12.2022 Gazette 2022/52)**

(54) **PROCEDE DE PREPARATION DE MERCAPTANS AVEC SULFHYDROLYSE DE DIALKYLSULFURES PURIFIES**

VERFAHREN ZUR HERSTELLUNG VON MERCAPTANEN MIT SULFHYDROLYSE VON GEREINIGTEN DIALKYLSULFIDEN

METHOD FOR PREPARING MERCAPTANS WITH SULFHYDROLYSIS OF PURIFIED DIALKYL SULFIDES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **21.06.2021 FR 2106568**

(43) Date de publication de la demande:
**01.05.2024 Bulletin 2024/18**

(73) Titulaire: **ARKEMA FRANCE**
**92800 Puteaux (FR)**

(72) Inventeurs:
• **FREMY, Georges**
**64170 LACQ (FR)**
• **SALEMBIER, Hélori**
**64170 LACQ (FR)**
• **LAUDUMIEY, Guillaume**
**64170 LACQ (FR)**
• **LACAMBRA, Joaquin**
**LACQ 64170 (FR)**
• **LAMANT, Eric**
**64170 LACQ (FR)**

(74) Mandataire: **Arkema Patent**
**Arkema France**
**DRD-DPI**
**51, Esplanade du Général de Gaulle**
**CS 10478**
**92907 Paris La Défense Cedex (FR)**

(56) Documents cités:
**FR-A1- 3 101 631**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**(Cont. page suivante)**

(52) Classification Coopérative des Brevets (CPC):
(Cont.)

C-Sets
**C07C 319/06, C07C 321/04;**
**C07C 319/28, C07C 321/04**

(52) Classification Coopérative des Brevets (CPC):
(Cont.)

C-Sets

**Description**

La présente invention concerne un procédé de préparation de mercaptans, à partir d'au moins un alcool et d'hydrogène sulfuré, intégrant un procédé de sulfhydrolyse de dialkylsulfures purifiés.

Les mercaptans présentent un grand intérêt industriel et sont aujourd'hui très largement utilisés par les industries chimiques, notamment comme matières premières pour la synthèse de molécules organiques plus complexes. Par exemple, le méthylmercaptan ($CH_3SH$) est utilisé comme matière première dans la synthèse de la méthionine, acide aminé essentiel pour l'alimentation animale. Le méthylmercaptan est également utilisé dans la synthèse des disulfures de dialkyles, en particulier dans la synthèse du disulfure de diméthyle (DMDS), additif de sulfuration de catalyseurs d'hydrotraitement de coupes pétrolières, entre autres applications.

Le document FR 3101631 décrit un procédé de préparation de méthylmercaptan.

La synthèse industrielle des mercaptans, et en particulier du méthylmercaptan, se fait généralement selon un procédé connu, à partir d'un alcool et d'hydrogène sulfuré à température élevée en présence d'un catalyseur selon l'équation (1) suivante :

Réaction principale

$$ROH + H_2S \rightarrow RSH + H_2O \qquad (1)$$

Cependant, cette réaction donne lieu à la formation de sous-produits, tels que les dialkylsulfures (qui sont dans le cas ci-dessous symétriques), selon l'équation (2) suivante :

$$ROH + RSH \rightarrow RSR + H_2O \qquad (2)$$

De plus, lorsque la réaction principale est effectuée en présence de plusieurs alcools, on peut également obtenir des dialkylsulfures dissymétriques selon les équations (3) et (4) suivantes (exemple donné avec deux alcools) :

$$ROH + R'OH + 2H_2S \rightarrow RSH + R'SH + 2H_2O \qquad (3)$$

$$ROH + R'SH \rightarrow RSR' + H_2O \qquad (4)$$

Les dialkylsulfures sous-produits, symétriques ou non, sont obtenus en grande quantité au niveau industriel et sont principalement amenés à être détruits. Ceci représente une perte d'efficacité pour le procédé de production des mercaptans et un coût supplémentaire lié à leur destruction.

Les dialkylsulfures sont parfois valorisés pour obtenir les mercaptans correspondants, grâce à la réaction (5) suivante (également appelée sulfhydrolyse) :

Réaction de sulfhydrolyse

$$RSR' + H_2S \rightarrow RSH + R'SH \qquad (5)$$

Dans le cas du méthylmercaptan, la réaction de sulfhydrolyse s'écrit selon l'équation (6) suivante :

$$CH_3SCH_3 + H_2S \rightarrow 2\ CH_3SH \qquad (6)$$

On peut ainsi intégrer une unité de sulfhydrolyse dans une unité de production principale de mercaptans obtenus à partir d'au moins un alcool et d'$H_2S$. Toutefois, l'ajout d'une nouvelle unité à une unité industrielle préexistante peut révéler des problèmes techniques d'intégration. En particulier, les présents inventeurs ont observé de façon surprenante des phénomènes de bouchage dans le réacteur ou en aval de l'unité de sulfhydrolyse, lorsque cette dernière est intégrée à une unité de production de mercaptans principale.

Aussi, il existe un besoin pour une unité de production de mercaptans à partir d'alcool(s) et d'$H_2S$ intégrant une réaction de sulfhydrolyse qui soit industriellement viable, sûre et peu coûteuse.

Un objectif de la présente invention est de fournir un procédé de préparation intégré de mercaptans, notamment à partir d'au moins un alcool et d'$H_2S$, dans lequel les dialkylsulfures sous-produits sont traités par sulfhydrolyse, de façon industriellement viable et sûre.

Un autre objectif de la présente invention est de fournir un procédé de préparation de mercaptans, notamment à partir d'au moins un alcool et d'$H_2S$, intégrant un procédé de sulfhydrolyse facile à mettre en œuvre, en particulier à l'aide d'installations simples et peu coûteuses.

En effet, il a été observé que la préparation de mercaptan(s) à partir d'au moins un alcool et d'$H_2S$ pouvait conduire à la formation d'impuretés dialkyldisulfures (notées DADS et de type R-S-S-R). Sans vouloir être lié par la théorie, ces DADS pourraient se former selon l'équation bilan suivante (7) (exemple à partir du méthanol) :

$$2CH_3SH + CH_3OH \rightarrow CH_3\text{-}S\text{-}S\text{-}CH_3 + CH_4 + H_2O \qquad (7)$$

Les présents inventeurs ont déterminé que ces DADS se retrouvent avec le(s) dialkylsulfure(s) et donc ensuite dans le réacteur où s'effectue la sulfhydrolyse. Ils peuvent alors conduire au cours du temps à des pertes de charges sur le catalyseur et/ou à des bouchages au niveau de ce réacteur ou plus en aval dans le procédé. Ce phénomène pourrait s'expliquer par un cokage du catalyseur lié à des réactions parasites ou secondaires de la réaction de sulfhydrolyse avec les DADS. Les produits ou impuretés soufrés formés par de telles réactions peuvent s'accumuler et créer des bouchages au niveau des installations industrielles, engendrant des problèmes de sécurité et de production évidents. Ceci peut être d'autant plus problématique lorsque l'on souhaite recycler le flux sortant du réacteur de sulfhydrolyse dans l'unité de production principale de mercaptan(s).

En particulier, lorsque du méthylmercaptan est formé à partir de méthanol et d'$H_2S$, il peut se former secondairement du diméthyldisulfure (DMDS). Lorsque ce DMDS est présent lors de la réaction de sulfhydrolyse, il a été observé des bouchages par des impuretés soufrées des installations (du réacteur et en aval du réacteur de sulfhydrolyse).

Les présents inventeurs ont découvert de façon surprenante, que lorsqu'on effectue la sulfhydrolyse d'un dialkylsulfure préalablement séparé des DADS, ces phénomènes de pertes de charge et/ou de bouchages ne sont plus observés.

Ainsi, la présente invention concerne un procédé de préparation, de préférence en continu, d'au moins un mercaptan comprenant les étapes suivantes :

A) dans un premier réacteur, on introduit de l'$H_2S$ et au moins un alcool ;
B) on fait réagir l'$H_2S$ et ledit au moins un alcool pour obtenir un flux sortant comprenant au moins un mercaptan, au moins un dialkylsulfure et au moins un dialkyldisulfure (DADS) et éventuellement de l'$H_2S$ n'ayant pas réagi ;
C) on sépare dudit flux sortant de l'étape B) :

- un flux F1 comprenant le(s) mercaptan(s),
- un flux F2 comprenant le(s) dialkylsulfure(s) et le(s) DADS, et
- éventuellement un flux F3 comprenant de l'$H_2S$ ;

D) on effectue une étape de purification du flux F2 de façon à séparer :

- un flux F2' comprenant le(s) dialkylsulfure(s) ; et
- le(les) DADS(s) ;

E) dans un second réacteur, on introduit le flux F2' avec de l'$H_2S$ ;
F) on effectue une réaction de sulfhydrolyse du(des) dialkylsulfure(s) par l'$H_2S$ pour obtenir un flux F4 sortant comprenant le(s)dit(s) mercaptan(s), et éventuellement de l'$H_2S$ n'ayant pas réagi ;
G) éventuellement on recycle le flux F4 issu de l'étape F) à l'étape A).

_Sulfures, disulfures et mercaptans_

On entend notamment par sulfure, tout composé organique comprenant une fonction -C-S-C. On entend notamment par disulfure, tout composé organique comprenant une fonction -C-S-S-C.

On entend notamment par dialkylsulfure, un composé de formule générale (I) suivante :

$$R\text{-}S\text{-}R' \qquad (I)$$

dans laquelle, R et R', identiques ou différents, sont indépendamment l'un de l'autre un radical hydrocarboné, saturé, linéaire, ramifié ou cyclique, éventuellement substitué.

De préférence, R et R', identiques ou différents, sont indépendamment l'un de l'autre un radical alkyle, linéaire, ramifié ou cyclique contenant entre 1 et 18 atome(s) de carbone, de préférence entre 1 et 12 atome(s) de carbone.

R et R', identiques ou différents, peuvent être choisis indépendamment l'un de l'autre parmi le groupe constitué du méthyle, de l'éthyle, du propyle, du butyle, du pentyle, de l'hexyle, de l'heptyle, de l'octyle, du nonyle, du décyle, de l'undécyle et du dodécyle (ainsi que leurs isomères de position). De préférence, R et R', identiques ou différents, peuvent être choisis indépendamment l'un de l'autre parmi le groupe constitué du méthyle, de l'éthyle, de l'octyle, et du dodécyle.

De préférence, R et R' sont identiques (ce qui correspond à un dialkylsulfure symétrique). Les dialkylsulfures symétriques sont en particulier de formule générale (II) suivante :

R-S-R          (II)

dans laquelle R est défini tel que ci-dessus.

En particulier, les dialkylsulfures selon l'invention sont choisis parmi le groupe constitué du diméthylsulfure, du diéthylsulfure, du dioctylsulfure, du didodécylsulfure et du méthyléthylsulfure. Les dialkylsulfures selon l'invention peuvent être choisis parmi le groupe constitué du diméthylsulfure, di-n-propylsulfure, di-isopropylsulfure, di-n-butylsulfure, di-sec-butylsulfure et du di-isobutylsulfure. De façon tout particulièrement préférée, le dialkylsulfure est le diméthylsulfure (DMS).

Les mercaptans selon l'invention sont notamment ceux correspondant à la sulfhydrolyse des dialkylsulfures tels que définis ci-dessus. On entend notamment par mercaptans, les alkylmercaptans.

En particulier, on entend par alkylmercaptan, un composé de formule générale (III) ou (IV) suivante :

R-SH          (III)

ou

R'SH          (IV),

dans lesquelles R et R' sont tels que définis pour la formule générale (I) ci-dessus.

De façon particulièrement préférée, le mercaptan est le méthylmercaptan.

En particulier, on entend par dialkyldisulfure (également appelés ci-après DADS), un composé de formule générale (V) suivante :

R-S-S-R'          (V),

dans laquelle R et R' sont tels que définis pour la formule générale (I) ci-dessus.

En particulier, les dialkyldisulfures selon l'invention sont choisis parmi le groupe constitué du diméthyldisulfure, du diéthyldisulfure, du dioctyldisulfure, du didodécyldisulfure et du méthyléthyldisulfure. Les dialkyldisulfures selon l'invention peuvent être choisis parmi le groupe constitué du diméthyldisulfure, du diéthyldisulfure, du dioctyldisulfure et du didodécyldisulfure. De façon tout particulièrement préférée, le dialkyldisulfure est le diméthyldisulfure (DMDS).

Pour les étapes A) et E), il est entendu que lorsque deux réactifs sont introduits dans un réacteur, ils peuvent être introduits chacun séparément dans le réacteur ou être combinés avant leur entrée dans le réacteur. L'introduction se fait de façon classique.

Le(s) réacteur(s) où se déroule(ent) la réaction principale et/ou la réaction de sulfhydrolyse peuvent être alimentés en $H_2S$ frais et/ou en $H_2S$ recyclé. L'$H_2S$ recyclé peut être l'$H_2S$ n'ayant pas réagi et récupéré à l'issue de l'une ou de plusieurs des étape(s) B), C), D) et/ou F), de préférence à l'issue des étapes C) et/ou F).

*Etape B) - Réaction entre au moins un alcool et l'$H_2S$ pour former un mercaptan*

La réaction entre un alcool et l'$H_2S$ pour former un mercaptan et de l'eau est une réaction connue, décrite par exemple dans les brevets US 2820062, US 7645906B2 et US 2820831. Par exemple, la réaction peut s'effectuer à une température comprise entre 200°C et 450°C et/ou à une pression allant d'une pression réduite à 100 bars. Généralement, un catalyseur est présent tel qu'une alumine promue par des métaux alcalins et/ou des métaux alcalinoterreux. L'$H_2S$ peut être présent en excès.

Parmi les réactifs, au moins un alcool, de préférence un ou deux alcool(s), peut(peuvent) être utilisé(s). Préférentiellement, un seul alcool est utilisé. Le(s) alcool(s) peuvent être choisis parmi les alcano-alcools, notamment ceux en $(C_1-C_{18})$, voire en $(C_1-C_{12})$, et leurs mélanges. En particulier, les alcools peuvent être choisis parmi le groupe constitué du méthanol, de l'éthanol, de l'octanol, du dodécanol et leurs mélanges. De préférence, l'alcool utilisé est le méthanol.

A l'issue de cette étape, on récupère notamment un flux sortant comprenant au moins un mercaptan, au moins un dialkylsulfure (en tant que sous-produit) et au moins un dialkyldisulfure (DADS) (en tant que sous-produit ou impureté) et éventuellement de l'$H_2S$. Le flux sortant peut également comprendre de l'eau.

*Etape C) - Séparation du(des) mercaptan(s) d'une part et du(des) dialkylsulfure(s) et DADS d'autre part*

Préalablement à l'étape C), le flux sortant issu de l'étape B) peut subir une ou plusieurs étapes de purification, par exemple de façon à éliminer l'eau et/ou l'$H_2S$ éventuellement présents. Cette(ces) étape(s) de purification peuvent se faire par séparation et/ou décantation et/ou distillation de façon classique.

A l'étape C), on sépare du flux sortant issu de l'étape B) :

- un flux F1 comprenant le(s) mercaptan(s),
- un flux F2 comprenant le(s) dialkylsulfure(s) et le(s) DADS, et
- éventuellement un flux F3 comprenant de l'$H_2S$.

L'étape de séparation C) peut se faire par des méthodes classiques, de préférence par distillation (notamment sous pression réduite). Lors de la distillation, la pression peut être comprise entre 1 et 40 bar absolus et/ou la température peut être comprise entre 20°C et 100°C en tête de colonne, et entre 40°C et 200°C en fond de colonne. Par exemple, la distillation peut se faire à une pression comprise entre 0,1 bar et 10 bars absolus, notamment entre 1 et 10 bars absolus.

De préférence, les flux F1 et F2 sont liquides et/ou le flux F3 est gazeux. Le flux F3 peut être en totalité ou en partie :

- recyclé vers l'étape A) et/ou
- recyclé à l'étape E) et/ou
- combiné avec le flux F1 ou F2.

*Etape D) - Purification des dialkylsulfures*

A l'étape D), on effectue notamment une étape de purification du flux F2 de façon à obtenir :

- un flux F2' comprenant le(s) dialkylsulfure(s) ; et
- le(s) DADS(s) ou un flux F5 comprenant le(s) DADS(s).

L'étape D) est notamment une étape de séparation d'une part du(des) dialkylsulfure(s) et d'autre part du(des) DADS et éventuellement des impuretés lourdes présentes dans le flux F2. L'étape D) est plus particulièrement une étape de séparation du diméthylsulfure du DMDS présent dans le flux F2.

On obtient notamment un flux F2' enrichi en dialkylsulfure(s). Les DADS ou le flux F5 peuvent être récupérés et éventuellement purifiés. On entend notamment par « flux F2' enrichi en dialkylsulfure(s) » un flux qui comprend un pourcentage en poids de dialkylsulfure(s) (par rapport au poids total dudit flux F2') supérieur au pourcentage en poids de dialkylsulfure(s) par rapport au poids total dudit flux avant ladite étape de purification (soit du flux F2).

Le flux F2 peut comprendre au moins 80%, de préférence au moins 95%, en poids de dialkylsulfure(s) par rapport au poids total du flux F2. Par exemple, le flux F2 comprend entre 95% et 99,9% en poids de dialkylsulfure(s) par rapport au poids total du flux F2.

Le flux F2 peut comprendre entre 0,1% et 20%, de préférence entre 0,1% et 5%, en poids de DADS par rapport au poids total du flux F2.

Ladite étape de purification peut correspondre à au moins une étape de distillation, ou à au moins une étape d'adsorption du(des) DADS(s) sur un support poreux (par exemple sur du charbon actif), ou à au moins une étape d'extraction sélective du(des) DADS(s) en utilisant un solvant non miscible avec le(s)dit(s) dialkylsulfure(s) et miscible avec le(s)dit(s) DADS(s), par exemple l'eau. Ces différentes techniques peuvent être combinées entre elles.

De façon tout à fait préférée, ladite étape de purification correspond à au moins une étape de distillation, préférentiellement à une seule étape de distillation. Selon un mode de réalisation, ladite étape de purification consiste en une seule étape de distillation.

La pression lors de la distillation peut être comprise entre 0,05 et 75 bars absolus, de préférence entre 1 et 30 bars absolus, plus particulièrement entre 5 et 15 bars absolus, par exemple à environ 10, 11, 12, 13, 14 ou 15 bars absolus.

La température de distillation peut être comprise entre 20°C et 250°C, de préférence entre 60°C et 200°C, plus préférentiellement entre 100°C et 180°C.

La température en tête de colonne peut être comprise entre 20°C et 250°C, de préférence entre 60°C et 200°C, plus préférentiellement entre 100°C et 180°C. En particulier, la température en tête de colonne est comprise entre 100°C et 180°C et à une pression comprise entre 5 et 15 bars absolus.

La température en pied de colonne peut être comprise entre 50°C et 300°C, de préférence entre 100°C et 250°C. En particulier, la température en pied de colonne est supérieure à la température de tête de colonne.

Une partie du flux F2' peut être renvoyée comme reflux dans la colonne de distillation (flux F6 ci-après). Le taux de reflux massique (F6/F2') dans la colonne peut être compris entre 0 et 0,99, de préférence entre 0 et 0,70.

De préférence, le flux F2' est récupéré en tête de colonne et les DADS (ou le flux F5) sont récupérés en pied de colonne.

Comme indiqué plus haut, le flux F3 peut être combiné avec le flux F2, et dans ce cas, il peut subir l'étape D) de purification. Dans le cas de la distillation, l'H$_2$S se retrouve en tête avec le flux F2' et peut être envoyé avec dans le réacteur de sulfhydrolyse.

La distillation peut être réalisée dans n'importe quel type de colonne à distiller connu. Il peut s'agir d'une colonne à plateaux (par exemple plateaux à calottes, plateaux à soupapes ou plateaux perforés) ou à garnissage (par exemple à garnissage en vrac ou structuré). La distillation peut être réalisée dans une colonne à plateaux, de préférence comprenant entre 5 et 50 plateaux, plus préférentiellement entre 10 et 40 plateaux, par exemple entre 10 et 30 plateaux. La distillation peut également être effectuée dans une colonne à cloison (appelée DWC en langue anglaise pour Divided Wall Column). La cloison peut être fixe ou mobile, par exemple avec un garnissage structuré ou vrac.

A l'issue de l'étape D), le flux F2' comprend notamment moins de 1000 ppm (massique), de préférence moins de 500 ppm, plus préférentiellement moins de 100 ppm voire moins de 10 ppm de DADS. En particulier, le flux F2' comprend strictement moins de 1000 ppm (massique). On peut récupérer le(s) mercaptan(s) au niveau du flux F1 et/ou du flux F4, de préférence par récupération du flux F1.

_Etape F) - Réaction de sulfhydrolyse du(des) dialkylsulfure(s) par l'H$_2$S_

Dans un second réacteur, on introduit le flux F2' avec un flux d'H$_2$S.

Les réactifs de la sulfhydrolyse peuvent être à l'état gazeux, liquide ou solide, de préférence gazeux ou liquide, aux conditions de température et de pression de la réaction.

La température de réaction de la sulfhydrolyse peut être comprise entre 100°C et 500°C, de préférence entre 200°C et 400°C, de préférence encore entre 200°C et 380°C, plus préférentiellement entre 250°C et 380°C.

La réaction de sulfhydrolyse peut être réalisée à une pression comprise entre 50 mbar et 100 bar absolus, de préférence entre la pression atmosphérique (environ 1 bar) et 50 bars absolus, et avantageusement entre 5 et 20 bars absolus.

Le ratio molaire H$_2$S/dialkylsulfure peut être compris entre 0,1/1 et 50/1, de préférence entre 2/1 et 20/1. De préférence ledit ratio est compris entre 2/1 et 15/1, de préférence encore entre 2/1 et 8/1, par exemple entre 2/1 et 6/1, tel que 4/1.

Le débit du dialkylsulfure dans le réacteur où se déroule la sulfhydrolyse peut être progressif. La réaction s'effectue avantageusement en présence d'un catalyseur. Les réactifs (dialkylsulfure(s) et H$_2$S) peuvent respecter un temps de contact particulier avec ce dernier. Ce paramètre est exprimé avec l'équation de la vitesse volumique horaire :

(WH) = (débit volumique total gazeux CNTP de dialkylsulfure + H$_2$S entrant) / (Volume de catalyseur dans le réacteur).

La VVH peut être comprise entre 100 et 1200 h$^{-1}$.

La GHSV (pour Gas Hourly Space Velocity en langue anglaise) peut être compris entre 1 et 100 000 h$^{-1}$, de préférence entre 100 et 10 000 h$^{-1}$, plus préférentiellement entre 100 et 3000 h$^{-1}$.

La réaction de sulfhydrolyse peut se dérouler dans tout type de réacteur, par exemple des réacteurs tubulaires à lit fixe, multitubulaires, à micro-canaux, à paroi catalytique ou à lit fluidisé, de préférence un réacteur tubulaire à lit fixe.

La quantité de chaque réactif fourni au réacteur peut varier en fonction des conditions de réaction (par exemple, la température, la vitesse volumique horaire, etc.) et se détermine selon les connaissances classiques. L'hydrogène sulfuré peut être présent en excès.

Tout type de catalyseur permettant de catalyser la réaction de sulfhydrolyse peut être utilisé. On peut notamment citer les catalyseurs, promus ou non, à base de zéolithes, d'alumine (Al$_2$O$_3$), de silice (SiO$_2$), de dioxyde de titane (TiO$_2$), d'aluminosilicate, de bentonite ou de zircone (ZrO$_2$). Ces catalyseurs comprennent ou peuvent être constitué(s) de zéolithes, d'alumine (Al$_2$O$_3$), de silice (SiO$_2$), de dioxyde de titane (TiO$_2$), d'aluminosilicate, de bentonite ou de zircone (ZrO$_2$) et éventuellement d'un ou plusieurs promoteur(s).

On entend notamment par _« promoteur »_ (également appelé _« dopant »_) une substance chimique ou une composition de substances chimiques pouvant modifier, notamment améliorer, l'activité catalytique d'un catalyseur. Par exemple, on entend par _« promoteur »,_ une substance chimique ou une composition de substances chimiques permettant d'améliorer la conversion et/ou la sélectivité de la réaction catalysée par rapport au catalyseur seul. De telles substances sont connues, par exemple les métaux alcalins, le Nickel (Ni), le Molybdène (Mo), le Cobalt (Co), le Tungstène (W) ou leurs combinaisons (par exemple les combinaisons NiMo et CoMo). Il est entendu que ces promoteurs peuvent être sous leur forme oxyde ou sulfurée (par exemple le sodium peut être sous la forme oxydée Na$_2$O). De préférence, le promoteur est choisi parmi les oxydes de métaux alcalins, en particulier Na$_2$O.

On entend notamment par métal alcalin, le lithium, le sodium, le potassium, le rubidium et le césium, de préférence le sodium.

En particulier, ledit catalyseur comprend moins de 10% en poids de promoteur, plus préférentiellement moins de 2% en poids de promoteur, par rapport au poids total du catalyseur. Ledit catalyseur peut comprendre entre 0% et 10% en poids de promoteur, de préférence entre 0% et 2% en poids de promoteur, par exemple entre 0,01% et 2% en poids de promoteur, par rapport au poids total dudit catalyseur.

On peut ainsi utiliser les catalyseurs suivants :

- les zéolithes promues ou non ; de préférence les zéolithes de type X , Y ou L, plus préférentiellement les zéolithes Y ;
- les catalyseurs à base d'alumine, promus ou non ;
  on peut citer en particulier les catalyseurs à base d'alumine, à base de NiMo (Nickel/Molybdène) et/ou CoMo (Cobalt/Molybdène) supportés sur alumine, à base de sulfure de cadmium supporté sur alumine, à base de trisulfure de tungstène supporté sur alumine, à base d'alumine promue par au moins 1% en poids d'oxyde de métal alcalin, ou encore à base d'alumine non promue, par exemple la gamma-alumine (de tels catalyseurs sont notamment décrits dans les demandes WO 2018/035316, WO 2017/210070 et US 2008/0200730) ;
- les catalyseurs à base de silice ($SiO_2$), promus ou non ;
- les catalyseurs à base de dioxyde de titane ($TiO_2$), promus ou non (notamment tels que décrits dans la demande FR3101631) ;
- les catalyseurs à base d'aluminosilicate, promus ou non ;
- les catalyseurs à base de bentonite, promus ou non, par exemple promus par au moins 1% en poids d'un oxyde de métal alcalin tels que décrits dans le document US 2008/0200730 ; et
- les catalyseurs à base de zircone, promus ou non (notamment tels que décrits dans la demande FR 3101631).

De préférence, le catalyseur selon l'invention est une zéolithe de type X, Y ou L, plus préférentiellement de type Y, promue ou non.

Une zéolithe est un cristal formé d'un squelette ou support microporeux d'aluminosilicate, dont les espaces vides connectés sont initialement occupés par des cations et des molécules d'eau. Les zéolithes selon l'invention présentent en particulier un paramètre de maille compris entre 24,30 et 24,70 Å et/ou un rapport Si/Al compris entre 2,5 et 15. On peut ainsi citer les zéolithes commercialisées par la société Axens sous la dénomination TCC101®.

En particulier, ladite zéolithe comprend moins de 10% en poids d'oxyde de métal alcalin, plus préférentiellement moins de 2% en poids d'oxyde de métal alcalin, par rapport au poids total de la zéolithe. En particulier, ledit oxyde de métal alcalin est l'oxyde de sodium ($Na_2O$).

De façon tout particulièrement préférée, ledit catalyseur est une zéolithe de type Y comprenant entre 0% et 10%, de préférence entre 0,01% et 10%, plus préférentiellement entre 0,01% et 2% en poids d'un oxyde de métal alcalin (de préférence $Na_2O$), par rapport au poids total de la zéolithe.

Le cation initial de la zéolithe, par exemple le sodium, peut avoir été remplacé en partie ou en totalité par au moins un autre cation selon des techniques connues pour les zéolithes, par exemple choisi parmi le groupe constitué de (à l'exception du cation à remplacer dans la liste ci-dessous) : H, Li, Na, K, Mg, Ca, Cs, Ba, La, Zr, Mo, W, Mn, Re, Fe, Ru, Co, Rh, Ni, Pd, Pt, Cu, Zn, Ag, Sn et Ga.

Ces échanges cationiques peuvent être effectués selon des techniques classiques.

Par exemple, les zéolithes peuvent être traitées sous forme ammonium. Il s'agit d'une calcination en présence de vapeur d'eau. La zéolithe sous forme ammonium ($NH_4+$) est mise ensuite sous forme protonique (H+) par chauffage. Le traitement à la vapeur d'eau hydrolyse les liaisons Si-O-Al. L'aluminium migre dans le volume microporeux sous forme de débris aluminiques. Simultanément à la création de ces espèces aluminiques ou silicoaluminiques une partie du réseau s'effondre créant ainsi une mésoporosité. Le silicium de ces parties du réseau est alors transporté vers les sites vacants par la vapeur d'eau. De telles techniques sont notamment expliquées dans la publication Christine E. A. Kirschhock Eddy J. P. Feijen Pierre A. Jacobs Johan A. Martens ; First published: 15 March 2008 https://doi.org/10.1002/9783527610044. hetcat0010 (Part 2. Preparation of Solid Catalysts; 2.3. Bulk Catalysts and Supports; 2.3.5 Hydrothermal Zeolite Synthesis).

Les catalyseurs selon l'invention peuvent comprendre des stabilisants et/ou des liants. Les stabilisants et les liants sont ceux classiquement utilisés dans le domaine des catalyseurs. Les catalyseurs peuvent être préalablement activés ou pré-traités.

*Etape G) - Eventuel recyclage*

On peut recycler partiellement ou entièrement dans le premier réacteur de l'étape A) le flux F4 sortant du second réacteur de l'étape F). L'$H_2S$ contenu dans le flux F4 peut être ainsi totalement ou partiellement recyclé. Un tel recyclage présente notamment l'avantage de n'avoir qu'une seule entrée d'$H_2S$ pour tout le procédé de production de mercaptans, à l'entrée du réacteur de sulfhydrolyse par exemple.

Ainsi, le procédé de sulfhydrolyse de dialkylsulfures purifiés intégré à une installation industrielle de production de mercaptans selon l'invention permet de retraiter efficacement les dialkylsulfures sous-produits en produits d'intérêt, de

recycler avantageusement l'$H_2S$ et d'éviter les phénomènes de bouchages et/ou de pertes de charge de façon à opérer en sécurité et en continu.

Les mercaptans produits seront le résultat de la réaction principale et de la réaction de sulfhydrolyse, ce qui augmente la productivité.

De plus, les mercaptans issus de la sulfhydrolyse et l'$H_2S$ n'ayant pas réagi peuvent être réintroduits directement (notamment sans étape de purification intermédiaire), dans le réacteur principal et ceci sans conséquence pour la réaction entre l'(les) alcool(s) et l'$H_2S$.

Les mercaptans produits par les deux réactions (principale et de sulfhydrolyse) peuvent alors être purifiés et/ou récupérés à un seul endroit, par exemple en sortie du réacteur principal.

Ainsi, selon l'invention, on peut obtenir un procédé de valorisation simple et efficace des dialkylsulfures qui soit totalement intégré dans une chaine de production industrielle de mercaptans. Ce dispositif est notamment de mise en œuvre simple : il peut se raccorder facilement à l'unité principale et ne requiert que peu de modifications sur cette dernière.

La présente invention concerne également l'utilisation d'au moins une distillation pour purifier un flux comprenant un dialkylsulfure de ses impuretés DADS, avant d'effectuer la sulfhydrolyse dudit dialkylsulfure par de l'$H_2S$, notamment telle que décrit ci-dessus.

L'expression « compris entre X et X » s'entend bornes incluses sauf mention particulière.

### Description des figures

**Figure 1** :

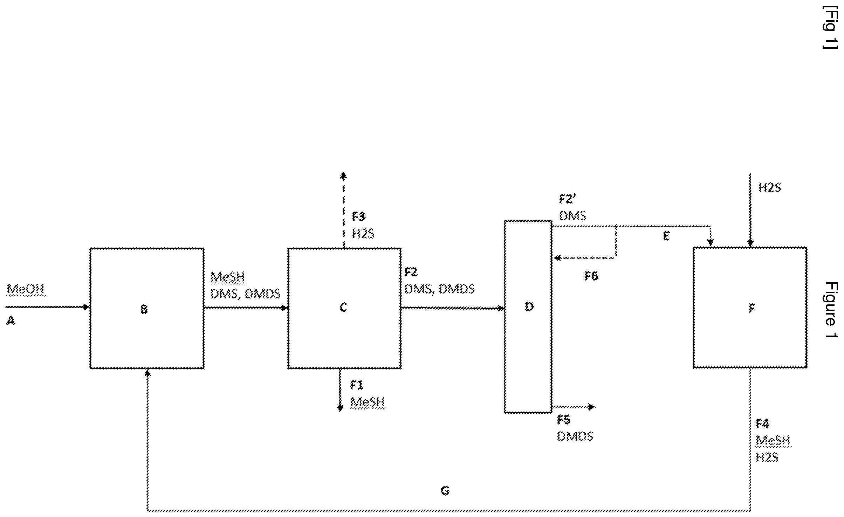

**La Figure 1** représente schématiquement une unité de production de méthylmercaptan intégrant un procédé de sulfhydrolyse d'un diméthylsulfure (DMS) purifié.

Lors de l'étape A), l'$H_2S$ et le méthanol sont introduits pour former dans le réacteur où se déroule l'étape B) un flux comprenant du méthylmercaptan, du DMS et du DMDS. Une séparation de ce flux est effectuée lors de l'étape C) pour obtenir :

- un flux F1 comprenant le méthylmercaptan,
- un flux F2 comprenant le DMS et le DMDS, et

- un flux optionnel F3 comprenant de l'$H_2S$.

Le flux F2 est distillé de façon à séparer le DMS de l'impureté DMDS et à obtenir un flux F2' en tête de colonne comprenant le DMS purifié. Un flux F5 comprenant le DMDS est obtenu en pied de colonne. Un flux F6, partie de F2', est renvoyé dans la colonne de distillation. Le flux F2' est introduit avec un flux d'$H_2S$, dans un réacteur pour effectuer la réaction de sulfhydrolyse (étape F)). On obtient un flux F4 sortant comprenant du méthylmercaptan et de l'$H_2S$. Le flux F4 est entièrement recyclé à l'étape A).

Les exemples suivants sont donnés à titre illustratif et ne sont pas limitatifs de la présente invention.

EXEMPLES

**Exemple 1** : **Séparation de l'impureté DMDS avant la réaction de sulfhydrolyse**

Essai A :

On réalise une réaction de sulfhydrolyse du diméthylsulfure (DMS) en présence ou en l'absence de DMDS, comme suit.

On introduit dans un réacteur du DMS comprenant (par rapport au poids total DMS+DMDS):

- soit 0,02% en poids de DMDS ;
- soit 14% en poids de DMDS.

On effectue la réaction de sulfhydrolyse dans les conditions suivantes.

Le catalyseur utilisé est le TCC101® d'Axens (catalyseur sous forme extrudée 1/8 avec un rayon interne de 7,7mm).

Il s'agit d'une zéolithe de type Y, ayant un paramètre de maille compris entre 24,30 et 24,70 Å, un rapport Si/Al compris entre 2,5 et 15 et comprenant moins de 10% en poids de $Na_2O$. La température de réaction est de 340 °C et la pression est de 25 barg.

Le ratio molaire $H_2S$/DMS est de 30,0.

Résultat : Avec un DMS comprenant 14% en poids de DMDS, on observe des phénomènes de bouchage dans le réacteur au bout de quelques heures tandis qu'avec un DMS comprenant 0,02% en poids de DMDS on n'observe aucun bouchage après 1000 heures.

Cet essai démontre le rôle de l'impureté DMDS dans les phénomènes de bouchage.

Essai B :

Avant de réaliser la réaction de sulfhydrolyse telle que décrite dans l'essai A, le DMS introduit est préalablement séparé ou non de l'impureté DMDS par distillation.

Les conditions de distillation sont les suivantes :

On utilise une colonne avec un nombre de plateaux compris entre 10 et 20.

La pression en tête de colonne est comprise entre 5 et 15 barg.

La température en tête de colonne est comprise entre 130°C et 140°C.

La température en pied de colonne est comprise entre 135°C et 150°C.

Le débit de reflux est compris entre 900 kg/h et 1200 kg/h.

Composition du flux entrant :

[Tableau 1]

| Composition massique du flux introduit dans le réacteur de sulfhydrolyse | Sans Distillation (F2) | Avec Distillation (F2') |
|---|---|---|
| %DMS | 98,8% | 99,3% |
| %MeSH | 0,07% | 0,08% |
| %$H_2O$ | 0,60% | 0,60% |
| %DMDS | 0,53% | 0,02% |

Sans distillation préalable, on observe un phénomène de bouchage après 100 h de mise en œuvre de la sulfhydrolyse. Avec distillation préalable, on n'observe aucun bouchage après 1000 h.

**Revendications**

1. Procédé de préparation d'au moins un mercaptan comprenant les étapes suivantes :

   A) dans un premier réacteur, on introduit de l'$H_2S$ et au moins un alcool ;
   B) on fait réagir l'$H_2S$ et ledit au moins un alcool pour obtenir un flux sortant comprenant au moins un mercaptan, au moins un dialkylsulfure et au moins un dialkyldisulfure (DADS) et éventuellement de l'$H_2S$ n'ayant pas réagi ;
   C) on sépare dudit flux sortant de l'étape B) :

       - un flux F1 comprenant le(s) mercaptan(s),
       - un flux F2 comprenant le(s) dialkylsulfure(s) et le(s) DADS, et
       - éventuellement un flux F3 comprenant de l'$H_2S$ ;

   D) on effectue une étape de purification du flux F2 de façon à séparer :

       - un flux F2' comprenant le(s) dialkylsulfure(s) ; et
       - le(les) DADS(s) ;

   E) dans un second réacteur, on introduit le flux F2' avec de l'$H_2S$ ;
   F) on effectue une réaction de sulfhydrolyse du(des) dialkylsulfure(s) par l'$H_2S$ pour obtenir un flux F4 sortant comprenant le(s)dit(s) mercaptan(s), et éventuellement de l'$H_2S$ n'ayant pas réagi ;
   G) éventuellement on recycle le flux F4 issu de l'étape F) à l'étape A).

2. Procédé de préparation selon la revendication 1, dans lequel ladite étape D) correspond à au moins une étape de distillation, ou à au moins une étape d'adsorption du(des) DADS sur un support poreux, ou à au moins une étape d'extraction sélective du(des) DADS en utilisant un solvant non miscible avec le(s)dit(s) dialkylsulfure(s) et miscible avec le(s)dit(s) DADS(s).

3.  Procédé de préparation selon la revendication 1 ou 2, dans lequel ladite étape D) correspond à au moins une étape de distillation, préférentiellement à une seule étape de distillation.

4.  Procédé de préparation selon la revendication 3, dans lequel lors de l'étape de distillation D), la pression est comprise entre 0,05 et 75 bars absolus, de préférence entre 1 et 30 bars absolus, plus particulièrement entre 5 et 15 bars absolus.

5.  Procédé de préparation selon la revendication 3 ou 4, dans lequel lors de l'étape de distillation D), une partie du flux F2' est renvoyée comme reflux dans la colonne de distillation.

6.  Procédé de préparation selon l'une quelconque des revendications 3 à 5, dans lequel lors de l'étape de distillation D) :

    - la température en tête de colonne est comprise entre 20°C et 250°C, de préférence entre 60°C et 200°C, plus préférentiellement entre 100°C et 180°C ; et
    - la température en pied de colonne est comprise entre 50°C et 300°C, de préférence entre 100°C et 250°C.

7.  Procédé de préparation selon l'une quelconque des revendications précédentes, dans lequel l'étape F) est réalisée en présence d'un catalyseur choisi parmi les catalyseurs, promus ou non, à base de zéolithes, d'alumine ($Al_2O_3$), de silice ($SiO_2$), de dioxyde de titane ($TiO_2$), d'aluminosilicate, de bentonite ou de zircone ($ZrO_2$), de préférence le catalyseur est une zéolithe.

8.  Procédé de préparation selon l'une quelconque des revendications précédentes, dans lequel lors l'étape F), le ratio molaire $H_2S$/dialkylsulfure est compris entre 0,1/1 et 50/1, de préférence entre 2/1 et 20/1, plus préférentiellement entre 2/1 et 8/1.

9.  Procédé de préparation selon l'une quelconque des revendications précédentes, dans lequel lors de l'étape G), la totalité du flux F4 est recyclée à l'étape A).

10. Procédé de préparation selon l'une quelconque des revendications précédentes, dans lequel l'alcool de l'étape A) est le méthanol.

**Patentansprüche**

1.  Verfahren zur Herstellung mindestens eines Mercaptans, das die folgenden Schritte umfasst:

    A) Eintragen von $H_2S$ und mindestens einem Alkohol in einen ersten Reaktor;
    B) Umsetzen des $H_2S$ und des mindestens einen Alkohols unter Erhalt eines Ausgangsstroms, der mindestens ein Mercaptan, mindestens ein Dialkylsulfid und mindestens ein Dialkyldisulfid (DADS) und gegebenenfalls nicht umgesetztes $H_2S$ umfasst;
    C) Trennen des Ausgangsstroms von Schritt B) in:

    - einen Strom F1, der das Mercaptan bzw. die Mercaptane umfasst,
    - einen Strom F2, der das Dialkylsulfid bzw. die Dialkylsulfide und das bzw. die DADS umfasst, und
    - gegebenenfalls einen Strom F3, der $H_2S$ umfasst;

    D) Durchführen eines Schritts der Reinigung des Stroms F2 zum Trennen in:

    - einen Strom F2', der das Dialkylsulfid bzw. die Dialkylsulfide umfasst; und
    - das bzw. die DADS;

    E) Eintragen des Stroms F2' mit $H_2S$ in einen zweiten Reaktor;
    F) Durchführen einer Sulfhydrolysereaktion mit $H_2S$ unter Erhalt eines Ausgangsstroms F4, der das Mercaptan bzw. die Mercaptane und gegebenenfalls nicht umgesetztes $H_2S$ umfasst;
    G) gegebenenfalls Rezyklieren des Stroms F4 aus Schritt F) in Schritt A).

**2.** Herstellungsverfahren nach Anspruch 1, wobei der Schritt D) mindestens einem Destillationsschritt oder mindestens einem Schritt der Absorption des bzw. der DADS an einem porösen Träger oder mindestens einem Schritt der selektiven Extraktion des bzw. der DADS unter Verwendung eines Lösungsmittels, das mit dem Dialkylsulfid bzw. den Dialkylsulfiden nicht mischbar und mit dem bzw. den DADS mischbar ist, entspricht.

**3.** Herstellungsverfahren nach Anspruch 1 oder 2, wobei der Schritt D) mindestens einem Destillationsschritt, vorzugsweise einem einzigen Destillationsschritt, entspricht.

**4.** Herstellungsverfahren nach Anspruch 3, wobei im Destillationsschritt D) der Druck zwischen 0,05 und 75 bar absolut, vorzugsweise zwischen 1 und 30 bar absolut, spezieller zwischen 5 und 15 bar absolut, liegt.

**5.** Herstellungsverfahren nach Anspruch 3 oder 4, wobei im Destillationsschritt D) ein Teil des Stroms F2' als Rücklauf in die Destillationssäule zurückgeführt wird.

**6.** Herstellungsverfahren nach einem der Ansprüche 3 bis 5, wobei im Destillationsschritt D):

- die Temperatur am Kopf der Säule zwischen 20 °C und 250 °C, vorzugsweise zwischen 60 °C und 200 °C, weiter bevorzugt zwischen 100 °C und 180 °C, liegt und
- die Temperatur am Boden der Säule zwischen 50 °C und 300 °C, vorzugsweise zwischen 100 °C und 250 °C, liegt.

**7.** Herstellungsverfahren nach einem der vorhergehenden Ansprüche, wobei Schritt F) in Gegenwart eines Katalysators durchgeführt wird, der aus promotierten oder nicht promotierten Katalysatoren auf Basis von Zeolithen, Aluminiumoxid ($Al_2O_3$), Siliciumdioxid ($SiO_2$), Titandioxid ($TiO_2$), Aluminosilikat, Bentonit oder Zirconiumdioxid ($ZrO_2$) ausgewählt wird, wobei es sich bei dem Katalysator vorzugsweise um einen Zeolith handelt.

**8.** Herstellungsverfahren nach einem der vorhergehenden Ansprüche, wobei in Schritt F) das $H_2S$/Dialkylsulfid-Molverhältnis zwischen 0,1/1 und 50/1, vorzugsweise zwischen 2/1 und 20/1, weiter bevorzugt zwischen 2/1 und 8/1, liegt.

**9.** Herstellungsverfahren nach einem der vorhergehenden Ansprüche, wobei in Schritt G) der gesamte Strom F4 in Schritt A) rezykliert wird.

**10.** Herstellungsverfahren nach einem der vorhergehenden Ansprüche, wobei es sich bei dem Alkohol von Schritt A) um Methanol handelt.

**Claims**

**1.** Process for preparing at least one mercaptan, comprising the following steps:

A) $H_2S$ and at least one alcohol are introduced into a first reactor;
B) the $H_2S$ and said at least one alcohol are reacted to obtain an outlet stream comprising at least one mercaptan, at least one dialkyl sulfide and at least one dialkyl disulfide (DADS) and possibly unreacted $H_2S$;
C) said outlet stream obtained from step B) is separated into:

- a stream F1 comprising the mercaptan(s),
- a stream F2 comprising the dialkyl sulfide(s) and the DADS(s), and
- optionally a stream F3 comprising $H_2S$;

D) a purification step is performed on stream F2 in order to separate:

- a stream F2' comprising the dialkyl sulfide(s); and
- the DADS(s);

E) stream F2' is introduced with $H_2S$ into a second reactor;
F) a sulfhydrolysis reaction of the dialkyl sulfide(s) with $H_2S$ is performed to obtain an outlet stream F4 comprising said mercaptan(s) and possibly unreacted $H_2S$;

G) optionally, stream F4 obtained from step F) is recycled into step A).

2. Preparation process according to Claim 1, in which said step D) corresponds to at least one distillation step, or to at least one step of adsorption of the DADS(s) on a porous support, or to at least one step of selective extraction of the DADS(s) using a solvent that is immiscible with said dialkyl sulfide (s) and miscible with said DADS(s).

3. Preparation process according to Claim 1 or 2, in which said step D) corresponds to at least one distillation step, preferentially to a single distillation step.

4. Preparation process according to Claim 3, in which, in the distillation step D), the pressure is between 0.05 and 75 bar absolute, preferably between 1 and 30 bar absolute, more particularly between 5 and 15 bar absolute.

5. Preparation process according to Claim 3 or 4, in which, in the distillation step D), part of stream F2' is returned to the distillation column as reflux.

6. Preparation process according to any one of Claims 3 to 5, in which, in the distillation step D):

- the column head temperature is between 20°C and 250°C, preferably between 60°C and 200°C, more preferentially between 100°C and 180°C; and
- the column bottom temperature is between 50°C and 300°C, preferably between 100°C and 250°C.

7. Preparation process according to any one of the preceding claims, in which step F) is performed in the presence of a catalyst chosen from promoted or nonpromoted catalysts based on zeolites, alumina ($Al_2O_3$), silica ($SiO_2$), titanium dioxide ($TiO_2$), aluminosilicate, bentonite or zirconia ($ZrO_2$) catalysts; preferably, the catalyst is a zeolite.

8. Preparation process according to any one of the preceding claims, in which, in step F), the $H_2S$/dialkyl sulfide mole ratio is between 0.1/1 and 50/1, preferably between 2/1 and 20/1, more preferentially between 2/1 and 8/1.

9. Preparation process according to any one of the preceding claims, in which, in step G), all of the stream F4 is recycled into step A).

10. Preparation process according to any one of the preceding claims, in which the alcohol of step A) is methanol.

[Fig 1]                                        Figure 1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 3101631
- US 2820062 A
- US 7645906 B2
- US 2820831 A
- WO 2018035316 A
- WO 2017210070 A
- US 20080200730 A